(19) **Europäisches Patentamt / European Patent Office / Office européen des brevets**

(11) **EP 4 138 090 A1**

(12) **EUROPEAN PATENT APPLICATION**
published in accordance with Art. 153(4) EPC

(43) Date of publication:
**22.02.2023 Bulletin 2023/08**

(21) Application number: **21939981.3**

(22) Date of filing: **23.11.2021**

(51) International Patent Classification (IPC):
**G16H 20/60** (2018.01)

(86) International application number:
**PCT/KR2021/017264**

(87) International publication number:
**WO 2022/270694 (29.12.2022 Gazette 2022/52)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **23.06.2021 KR 20210081845**
**08.09.2021 KR 20210119460**

(71) Applicant: **NUTRITIONCOURT**
**Seoul 06168 (KR)**

(72) Inventor: **LEE, Yea Hang**
**Gangnam-gu, Seoul 06168 (KR)**

(74) Representative: **Gulde & Partner**
**Patent- und Rechtsanwaltskanzlei mbB**
**Wallstraße 58/59**
**10179 Berlin (DE)**

(54) **PERSONALIZED HEALTHCARE SYSTEM AND OPERATION METHOD THEREOF**

(57)     The present invention relates to a customized healthcare system and an operation method thereof, and the operation method of the customized healthcare system according to an embodiment includes the steps of: acquiring at least one among health checkup information and medical treatment information of a user; extracting an analysis result for at least one test item on the basis of at least one among the health checkup information and the medical treatment information; determining a risk level of the user for the test item on the basis of the analysis result; selecting at least one among the recommended nutrients and/or the restricted nutrients for the user on the basis of the risk level; and providing customized nutritional information to the user on the basis of at least one among the recommended nutrients and the restricted nutrients, and the customized nutritional information includes information on at least one among the recommended nutrient, the restricted nutrient, a recommended food material, a recommended menu, a recommended meal pattern, a recommended diet, and a recommended health functional food.

[Figure 2]

# Description

## TECHNICAL FIELD

The present invention relates to a customized healthcare system and an operation method thereof.

## BACKGROUND ART

Interest in various nutrients that humans should take for life has become a particularly important issue to modern people, and in recent years, interest in nutrients necessary for human bodies is increasing in accordance with the trend that values quality of life.

In modern times, information on the nutrients is generally provided through Internet portal sites. However, nutrient information provided through Internet portal sites is nothing but general information common to a plurality of users, simply such as information on food materials or food, information on the nutrients contained in a corresponding food material or the like, information on the effects of corresponding food materials and/or nutrients (disease improvement, prevention effect, and the like), information on calorie, and the like.

Although the nutrients required for each user are absolutely different due to the user's individual health conditions, the risk level of a specific disease in the future based thereon, or the like, there is a limitation in that such universal and general nutritional information does not reflect the characteristics such as a disease risk level or the like of individual users at all. In addition, it is inconvenient in that users should search, confirm, and view by themselves what kind of nutrients they should take, what kind of food materials, diets, and health functional foods should be selected for this purpose, and what kind of nutrients should be taken cautiously.

Therefore, the need for new customized healthcare that can solve these conventional limitations and problems is gradually increasing.

## DISCLOSURE OF INVENTION

### TECHNICAL PROBLEM

Therefore, the present invention has been made in view of the above problems, and it is an object of the present invention to provide a customized healthcare system and an operation method thereof, which can select recommended nutrients and/or restricted nutrients on the basis of medical checkup information and medical treatment information, and provide optimal customized nutritional information to a user.

Another object of the present invention is to provide a customized healthcare system and an operation method thereof, which can provide a user with health information through a customized search that reflects individual health conditions.

### TECHNICAL SOLUTION

To accomplish the above objects, according to one aspect of the present invention, there is provided an operation method of a customized healthcare system, the method comprising the steps of: acquiring at least one among health checkup information and medical treatment information of a user; extracting an analysis result for at least one test item on the basis of at least one among the health checkup information and the medical treatment information; determining a risk level of the user for the test item on the basis of the analysis result; selecting at least one among the recommended nutrients and the restricted nutrients for the user on the basis of the risk level; and providing customized nutritional information to the user on the basis of at least one among the recommended nutrients and the restricted nutrients, wherein the customized nutritional information includes information on at least one among the recommended nutrient, the restricted nutrient, a recommended food material, a recommended menu, a recommended meal pattern, a recommended diet, and a recommended health functional food.

According to another aspect of the present invention, there is provided a customized healthcare system comprising: at least one memory for storing a program for performing customized healthcare; and at least one processor for acquiring at least one among health checkup information and medical treatment information of a user, extracting an analysis result for at least one test item on the basis of at least one among the health checkup information and the medical treatment information, determining a risk level of the user for the test item on the basis of the analysis result, selecting at least one among the recommended nutrients and/or the restricted nutrients for the user on the basis of the risk level, and providing customized nutritional information to the user on the basis of at least one among the recommended nutrients and the restricted nutrients by executing the program, wherein the customized nutritional information includes information on at least one among the recommended nutrient, the restricted nutrient, a recommended food material, a recommended menu, a recommended meal pattern, a recommended diet, and a recommended health functional food.

## BRIEF DESCRIPTION OF THE DRAWINGS

In order to further fully understand the drawings cited in the Detailed Description of the Invention, a brief description of each drawing is provided.

FIG. 1 is a view for explaining a customized healthcare system according to an embodiment of the present invention.

FIG. 2 is a sequence diagram illustrating an operation process of a customized healthcare system according to an embodiment of the present invention.

FIG. 3 is a flowchart illustrating an operation method of a customized healthcare system server according to an embodiment of the present invention.

FIG. 4 is a flowchart illustrating an embodiment of step S350 in FIG. 3.

FIG. 5 is a flowchart illustrating an embodiment of step S360 in FIG. 3.

FIG. 6 is a flowchart illustrating an embodiment of step S360 in FIG. 3.

FIG. 7 is a flowchart illustrating an embodiment of step S360 in FIG. 3.

FIG. 8 is a flowchart illustrating an operation method of a customized healthcare system server according to an embodiment of the present invention.

FIG. 9 is a view for explaining step S820 in FIG. 8.

FIG. 10 is a flowchart illustrating an embodiment of step S850 in FIG. 8.

FIG. 11 is a flowchart illustrating an embodiment of step S850 in FIG. 8.

FIGS. 12 to 15 are views for explaining an exemplary operation of a customized healthcare system according to an embodiment of the present invention.

FIG. 16 is a view for explaining an exemplary operation of a customized healthcare system according to an embodiment of the present invention.

FIG. 17 is a block diagram showing the configuration of a customized healthcare system according to an embodiment of the present invention.

## BEST MODE FOR CARRYING OUT THE INVENTION

Hereinafter, preferred embodiments of the present invention will be described in detail with reference to the accompanying drawings. At this point, it should be noted that the same components in the accompanying drawings are denoted by the same reference numerals as much as possible. In addition, detailed descriptions of well-known functions and configurations that may obscure the gist of the present invention will be omitted.

Some embodiments of the present invention may be represented by functional block components and various processing steps. Some or all of the functional blocks may be implemented in various numbers of hardware and/or software components that perform specific functions. For example, the functional blocks of the present invention may be implemented by one or more microprocessors or by circuit components for a predetermined function. In addition, for example, the functional blocks of the present invention may be implemented in various programming or scripting languages. The functional blocks may be implemented as an algorithm executed in one or more processors. In addition, the present invention may employ conventional techniques for the purpose of electronic environment setting, signal processing, and/or data processing.

In addition, the terms such as "...unit", "module", and the like described in this specification mean a unit that processes at least one function or operation, and may be implemented as hardware, software, or a combination of hardware and software. The "units" and "modules" are stored in an addressable storage medium and may be implemented by a program that can be executed by a processor.

For example, the "units" and "modules" may be implemented by components such as software components, object-oriented software components, class components, and task components, and processes, functions, attributes, procedures, subroutines, segments of program codes, drivers, firmware, micro codes, circuits, data, databases, data structures, tables, arrays, and variables.

Throughout the specification, when a part is "connected" to another part, it includes a case of being "indirectly connected" to another part with intervention of a device interposed therebetween, as well as a case of being "directly connected". Throughout the specification, when a part "includes" a certain component, it means that other components may be further included, rather than excluding other components, unless otherwise stated.

In addition, connection lines or connection members between the components shown in the drawings merely exemplify functional connections and/or physical or circuit connections. In an actual device, a connection between components may be shown by various replaceable or added functional connections, physical connections, or circuit connections.

The terms used in the present invention are used only to describe specific embodiments, and are not intended to limit the present invention. A singular expression includes a plural expression unless the context clearly dictates otherwise. It should be understood that in the present invention, the terms such as "include", "have", and the like are intended to designate presence of a feature, a number, a step, an operation, a component, a part, or a combination thereof described in the specification, and do not preclude the possibility of the presence or addition of one or more other features, numbers, steps, operations, components, parts, or combinations thereof. That is, the description of "including" a specific configuration in the present invention does not exclude configurations other than the corresponding configuration, and it means that additional configurations may be included in the embodiments of the present invention or the scope of the technical spirit of the present invention.

Some components of the present invention are not essential components for performing essential functions in the present invention, but may be optional components for merely improving performance. The present invention may be implemented by including only the components essential for implementing the essence of the present invention, excluding the components used only for performance improvement, and a structure including only the essential components, excluding the optional components used for improving performance, is also included in the scope of the present invention.

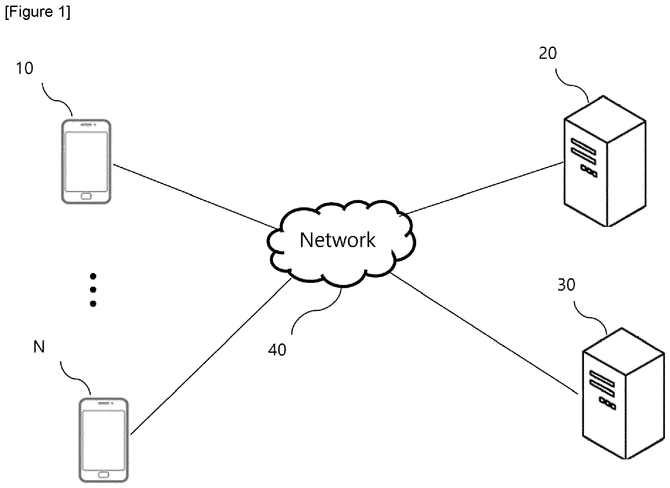

FIG. 1 is a view for explaining a customized healthcare system according to an embodiment.

Referring to FIG. 1, a customized healthcare system according to an embodiment may operate including terminals 10 to N, a customized healthcare system server 20, a database server 30, and a network 40.

The terminals 10 to N may include all types of devices that may access the network 40. For example, the terminals 10 to N may include a smart phone, a tablet computer, a PC, a notebook computer, a home appliance device, a medical device, a camera, a wearable device, and the like. In an embodiment, the terminals 10 to N may receive customized nutritional information from the customized healthcare system server 20.

In an embodiment, the terminals 10 to N may provide a predetermined user interface, receive personal information (identification information, age, gender, weight, allergy, medication, and the like) from a user through a user input, and transmit the personal information to the customized healthcare system server 20 and/or the database server 30 through the network 40.

The customized healthcare system server 20 provides computing resources, storage resources, and the like for providing customized nutritional information to a client through the network 40. Here, the client may include the terminals 10 to N. In an embodiment, the customized healthcare system server 20 may include various types of servers, such as an application server, a control server, a data storage server, a server for providing a specific function, and the like. In addition, the customized healthcare system server 20 may handle a process alone, or a plurality of servers may handle a process in cooperation.

In an embodiment, the customized healthcare system server 20 may acquire health condition information (for example, at least one among medical checkup information and medical treatment information) of a user, select recommended nutrients and/or restricted nutrients on the basis of the health condition, and provide customized nutritional information including at least one among a recommended nutrient, a restricted nutrient, a recommended food material, a recommended menu, a recommended meal pattern, a recommended diet, and a recommended health functional food to the terminal 10 on the basis of the amounts of the recommended nutrients and/or restricted nutrients.

In an embodiment, the customized healthcare system server 20 may acquire health condition information of a user, select recommended nutrients and/or restricted nutrients on the basis of the health condition, and create a customized database, and when the user inputs a search word (the name of a food, a food material, a health functional food, a nutrient, or the like) of a predetermined search target, the customized healthcare system server 20 may provide customized health information to the terminal 10 on the basis of the search word.

The database server 30 stores data needed for the operation of the customized healthcare system. In an embodiment, the database server 30 may be directly or indirectly operated and managed by at least one medical institution (hospital, examination institution, public health center, or the like) and/or national institution (e.g., National Health Insurance Service, Ministry of Food and Drug Safety, or the like) for maintenance of data, and may be configured to transfer corresponding data to a user or a business operator having a predetermined qualification. In an embodiment, the database server 30 may store medical checkup information, medical treatment information, and the like of a user. The information stored in the database server 30 may be transferred in response to a request of the customized healthcare system server 20 and used in the process of providing the customized nutritional information.

The network 40 may include all types of networks that the terminals 10 to N, the customized healthcare system server 20, and the database server 30 may access, such as the Internet, an intranet, an extranet, a local area network (LAN), a metropolitan area network (MAN), a wide area network (WAN), and the like.

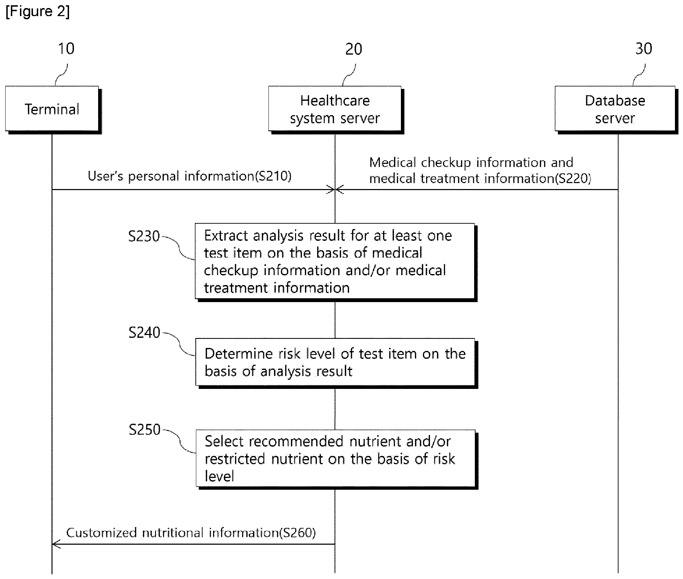

FIG. 2 is a sequence diagram illustrating an operation process of a customized healthcare system according to an embodiment.

Referring to FIG. 2, first, the customized healthcare system server 20 may receive personal information of a user from the terminal 10 at step S210, and receive health condition information of the user from the database server 30 at step 220. Here, the health condition information may include medical checkup information and/or medical treatment information. The medical checkup information may include information on a test result or an analysis result of at least one user for at least one test item of a test performed at a medical institution, and the medical treatment information may include diagnostic information or medication information that determines the health condition, presence of a disease, and the like of the user determined by the medical institution on the basis of the medical checkup information.

According to embodiments, only one of steps S210 and S220 may be performed.

Thereafter, at steps S230 and S240, the customized healthcare system server 20 extracts an analysis result for at least one test item on the basis of the medical checkup information and/or medical treatment information, and determines a risk level of the test item (or disease related thereto) on the basis of the analysis result. In an embodiment, the risk level may be indicated as a plurality of risk classes, such as normal, alert, risky, and the like.

At step S250, the customized healthcare system server 20 selects, on the basis of the risk level, at least one recommended nutrient for improving health risk factors related to the test item and at least one restricted nutrient to be avoided not to worsen the health risk factors.

Thereafter, at step S260, the customized healthcare system server 20 transmits customized nutritional information to the terminal 10 on the basis of the recommended nutrients and/or restricted nutrients. In an embodiment, the customized nutritional information may include information on at least one among a recommended nutrient, a restricted nutrient, a recommended food material containing a large number and/or amount of the recommended nutrients, a recommended menu, a recommended meal pattern, a recommended diet, and a recommended health functional food.

In an embodiment, the customized healthcare system server 20 may create a customized database by storing information on the recommended nutrients and/or restricted nutrients in correspondence to the user and the test items. At this point, corresponding information may be stored in the database server 30.

In an embodiment, the customized healthcare system server 20 may receive a customized search request for a search target from the terminal 10. At this point, the search target may be a food material, a nutrient, a food, and/or a health functional food, and the customized search request may include a search word corresponding to the search target. Thereafter, the customized healthcare system server 20 may detect health information customized to the user on the basis of the recommended nutrients and/or the restricted nutrients and the search word.

When the search target is a food, a food material, or a health functional food, the customized healthcare system server 20 may determine whether the search target is appropriate for the user by comparing the nutrients contained in the search target with the recommended nutrients and/or restricted nutrients.

Meanwhile, when the search target is a nutrient, the customized healthcare system server 20 may determine whether the search target is appropriate according to whether the nutrient corresponds to the recommended nutrients or the restricted nutrients. In addition, the customized healthcare system server 20 may detect at least one product containing the nutrient of the search target as a nutrient contained in the product, and determine whether each of the products is appropriate for the user by comparing the nutrients contained in each product with the recommended nutrients and/or the restricted nutrients.

Thereafter, the health information may be provided to the terminal 10 and displayed to the user. At this point, the health information may include basic information of the search target, nutrients contained therein, information on whether it is appropriate for the user, and the like.

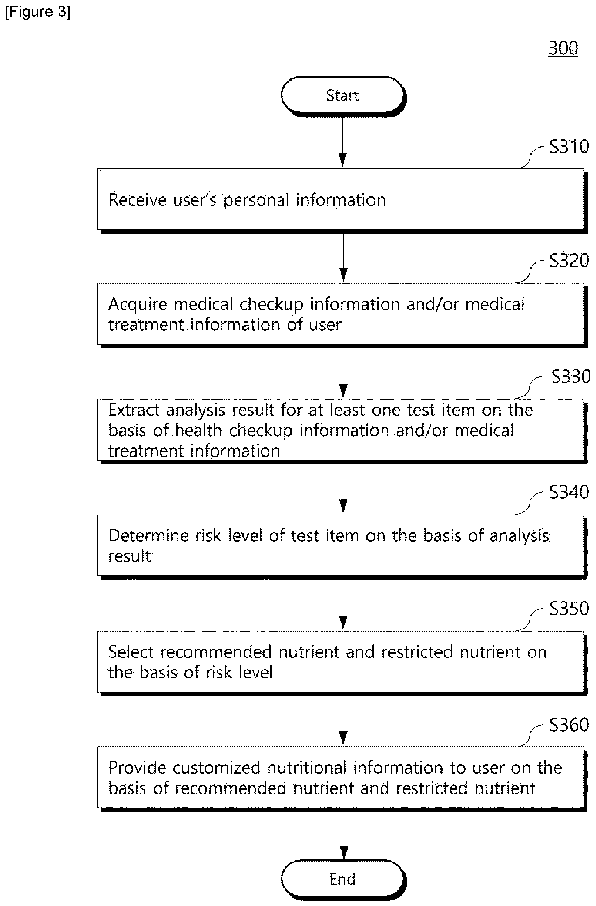

FIG. 3 is a flowchart illustrating an operation method of a customized healthcare system server according to an embodiment of the present invention.

At step S310, the customized healthcare system server 20 may receive personal information of a user from the terminal 10.

Here, the personal information may include information on at least one among the age, gender, weight, allergy, and medication of the user, as well as identification information (phone number, ID, name, date of birth, and the like) for identifying the user.

In order to perform step S310, the terminal 10 may be provided with a predetermined user interface for inputting personal information of the user. For example, the user may transfer the input personal information to the customized healthcare system server 20 by activating a corresponding user interface by driving a dedicated application installed in the terminal 10.

At step S320, the customized healthcare system server 20 may acquire health condition information of the user, i.e., at least one among health checkup information and medical treatment information. For example, the customized healthcare system server 20 may acquire medical checkup information and medical treatment information of the user from the database server 30 on the basis of the user's identification information. In an embodiment, the database server 30 may be directly or indirectly operated and managed by at least one medical institution (hospital, examination institution, public health center, or the like) and/or national institution (e.g., National Health Insurance Service, or the like) for maintenance of data.

The medical checkup information may include information on at least one user's test result for at least one test item of a test performed at a medical institution, and the medical treatment information may include diagnostic information or medication information that determines the health condition, presence of a disease, and the like of the user determined by the doctor or the like of a medical institution on the basis of the medical checkup information or the like.

In an embodiment, the medical checkup information may include information on a test result of at least one blood test item. Although the blood test item may include, for example, a blood sugar level, a cholesterol level, a hemoglobin level, a liver level, a kidney function level, and the like, it is not limited thereto.

In an embodiment, the customized healthcare system server 20 may further receive a medical questionnaire result of the user. The medical questionnaire result may be a result determined on the basis of a user's response to the medical questionnaire provided on the terminal 10. For example, the terminal 10 may display a medical questionnaire related to the user's health through a user interface, perform the medical questionnaire in a method of receiving a response to the medical questionnaire from the user, and transmit the result to the customized healthcare system server 20.

At steps S330 and S340, the customized healthcare system server 20 may extract an analysis result for at least one test item on the basis of at least one among the health checkup information and the medical treatment information, and determine a risk level of the user for the test item on the basis of the analysis result.

In an embodiment, the customized healthcare system server 20 may classify the risk level of the user for the test item (or a disease related to the test item) into a plurality of classes according to the analysis result. For example, the customized healthcare system server 20 may classify the risk level of the user into a plurality of risk classes, such as normal, alert, suspicious, and the like on the basis of numerical values measured for the test item.

In an embodiment, at step S340, in addition to the risk level for each of test items, a cross-risk level between the test items may be determined. For example, a risk level of "diabetes due to impaired liver function" may be determined by cross-analyzing a result of a first blood test for blood sugar and a result of a second blood test related to a liver level.

At step S350, the customized healthcare system server 20 may select at least one among recommended nutrients and restricted nutrients of the user on the basis of the determined risk level.

For example, the customized healthcare system server 20 may select at least one recommended nutrient beneficial for improving the test item, of which the risk level is determined to be higher than a predetermined level, and at least one restricted nutrient to be avoided not to worsen the test item.

At step S360, the customized healthcare system server 20 may provide customized nutritional information to the user on the basis of at least one among the recommended nutrients and the restricted nutrients.

In an embodiment, the customized nutritional information may include information on at least one among a recommended nutrient, a restricted nutrient, a recommended food material, a recommended menu, a recommended meal pattern, a recommended diet, and a recommended health functional food. For example, the customized healthcare system server 20 may receive information on a food ingredient table from a food ingredient DB or the like constructed in the database server 30, and provide the customized nutritional information to the user by selecting and transmitting a food material, a menu, a diet, a health functional food, or the like containing a lot of recommended nutrients and less or no restricted nutrients to the terminal 10 on the basis of information on the food ingredient table.

In an embodiment, the customized healthcare system server 20 may selectively provide at least one piece of customized nutritional information by assigning a predetermined priority on the basis of at least one among the number of recommended nutrients contained therein, the ratio of each recommended nutrient contained therein, and the amount of each recommended nutrient. For example, the customized healthcare system server 20 may be configured to preferentially provide the user with information on a food material or a health food within a predetermined ranking in order of the amount of the recommended nutrients contained therein, among a plurality of recommended food materials or recommended health functional foods.

In an embodiment, the operation method 300 may further include the step of providing, by the customized healthcare system server 20, purchase information for purchasing the recommended food material, recommended health functional food, and the like. Accordingly, the user may confirm various brands and/or vendors related to the recommended food material and the recommended health functional food through the terminal 10, and make a purchase of them.

In an embodiment, the operation method 300 may further include the step of collecting information on the consumption pattern of the user, by the customized healthcare system server 20, when a food material or a health functional food is ordered on the basis of the purchase information provided to the terminal 10. For example, the consumption pattern information may include data such as a user's actual purchase ratio, a list of actually purchased food materials or health functional foods, a time of intensive use by the user, and the like. In this case, the customized healthcare system server 20 may transmit the collected consumption pattern information to other customer companies (such as online shopping malls or the like) on the basis of the consent of the user, or analyze the user's consumption preference or the like by utilizing the consumption pattern information by himself or herself and provide customized advertisements or the like to the user.

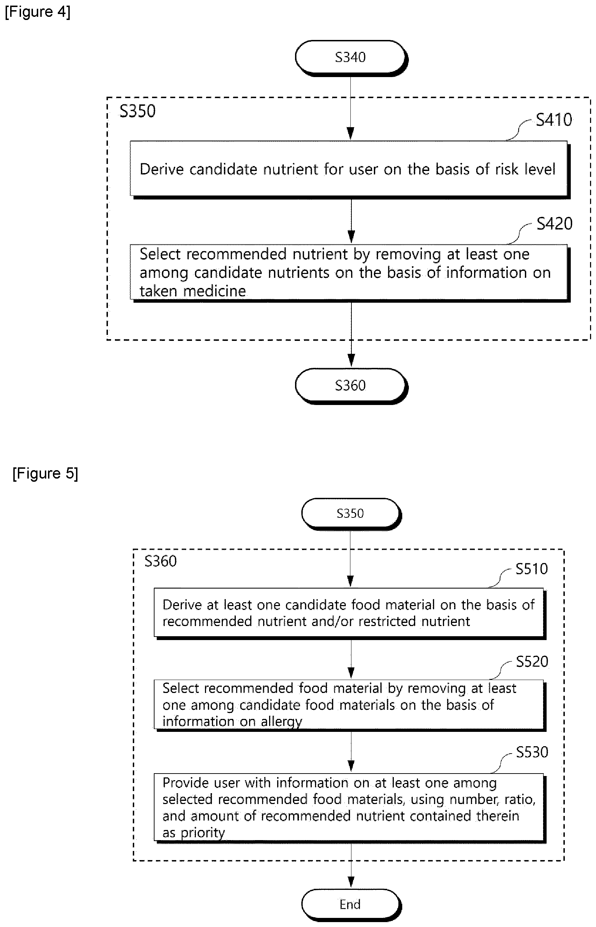

FIG. 4 is a flowchart illustrating an embodiment of step S350 in FIG. 3.

Referring to FIG. 4, the customized healthcare system server 20 may select a recommended nutrient by reflecting information on the medicine taken by the user.

First, at step S410, the customized healthcare system server 20 may derive at least one candidate nutrient for improving health risk factors related to the test item, on the basis of the risk level of the test item determined at step S340 of FIG. 4.

That is, for example, when it is determined that the risk of diabetes is high as the blood sugar level is high, candidate nutrients may be derived from at least one plant component for improving the risk of diabetes.

Subsequently, at step S420, the customized healthcare system server 20 may select a recommended nutrient by removing at least one candidate nutrient on the basis of the medicine taken by the user.

That is, since it is less needed to take additional nutrients overlapped with the nutrients included in the medicine currently taken by the user, the customized healthcare system server 20 may be configured to remove the overlapped nutrients among the derived candidate nutrients and finally select recommended nutrients.

FIG. 5 is a flowchart illustrating an embodiment of step S360 in FIG. 3.

Referring to FIG. 5, the customized healthcare system server 20 may select a recommended food material by reflecting information on allergies of the user.

First, at step S510, the customized healthcare system server 20 may derive at least one candidate food material on the basis of at least one among the recommended nutrients and the restricted nutrients selected through step S350 of FIG. 3. That is, the customized healthcare system server 20 may derive at least one candidate food material containing a large amount of recommended nutrients and less or no restricted nutrients.

Subsequently, at step S520, the customized healthcare system server 20 may select a recommended food material by removing at least one among the food materials on the basis of the allergy information. That is, the customized healthcare system server 20 may select a recommended food material by removing, among the candidate food materials, food materials that show an allergic reaction when the user takes.

Subsequently, at step S530, the customized healthcare system server 20 may provide the user with information on at least one among the selected recommended food materials, using at least one among the number of recommended nutrients contained therein, the ratio of each recommended nutrient contained therein, and the amount of each recommended nutrient as a priority.

For example, the customized healthcare system server 20 may be configured to preferentially provide the terminal 10 with information on the recommended food material within a predetermined ranking in order of the amount of the recommended nutrients contained therein.

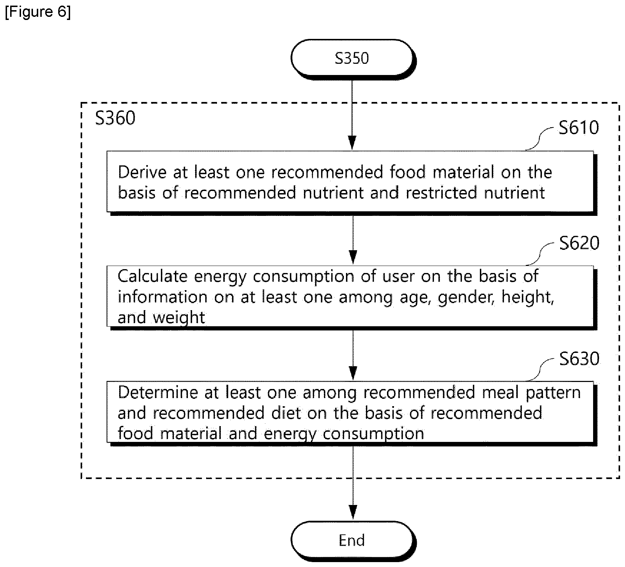

FIG. 6 is a flowchart illustrating an embodiment of step S360 in FIG. 3.

Referring to FIG. 6, the customized healthcare system server 20 may determine at least one among a recommended meal pattern and a recommended diet on the basis of energy consumption of the user.

First, at step S610, the customized healthcare system server 20 may derive at least one candidate food material on the basis of at least one among the recommended nutrients and the restricted nutrients selected through step S350 of FIG. 3.

Subsequently, at step S620, the customized healthcare system server 20 may calculate energy consumption of the user on the basis of information on at least one among the age, gender, height, and weight of the user included in the personal information of the user.

In an embodiment, the customized healthcare system server 20 may calculate a basal metabolic rate corresponding to the age, gender, height, and/or weight of the user, and in addition, it may calculate energy consumption of the user by reflecting the activity coefficient and digestive metabolic rate of the user.

For example, the energy consumption of the user may be calculated by the following equation.

$$\text{Energy consumption} = \text{Basal metabolic rate} \times \text{Activity coefficient} + \text{Digestive metabolic rate}$$

$$(\text{Digestive metabolic rate} = (\text{Basal metabolic rate} \times \text{Activity coefficient}) \times 0.1)$$

Here, the activity coefficient is an index indicating the level of user's activity. For example, the activity coefficient is divided into four stages of "inactive", "less active", "active", and "very active", and may be configured to have a different coefficient value in each stage. The activity coefficient may have a value in a predetermined range (e.g., a constant between 1 and 2), and may be configured to have a value increasing from the "inactive" stage toward to "active" stage. In addition, according to an embodiment, the activity coefficient may be configured to have a value that varies according to gender.

In an embodiment, the activity coefficient of the user may be calculated on the basis of personal information of the user. That is, when a questionnaire about the activity level of the user is provided to the terminal 10, and the user selects one among the four stages and transmits it to the customized healthcare system server 20 in response thereto, the activity coefficient may be calculated on the basis of the stage.

In an embodiment, at step S620, the energy consumption pattern of the user may be further calculated. For example, the customized healthcare system server 20 may further acquire information on the job, wake-up time and bedtime, exercise amount, and the like of the user through the terminal 10, and calculate a daily energy consumption pattern of the user on the basis of the acquired information.

Subsequently, at step S630, the customized healthcare system server 20 may determine at least one among the recommended meal pattern and the recommended diet on the basis of the energy consumption and/or energy consumption pattern of the user.

For example, the customized healthcare system server 20 may prepare a recommended diet mainly configured of foods containing food materials of high calorie while containing recommended nutrients for a user with high daily energy consumption. In addition, for example, in the energy consumption pattern of the user, a recommended diet for a user consuming much energy in the evening may be configured to include foods with high calorie in the dinner menu compared to those of users who do not consume much energy in the evening. In addition, for example, when it is determined good for a user to have two meals or four meals a day on the basis of the energy consumption and/or energy consumption pattern of the user, a recommended meal pattern of the user may be configured according thereto.

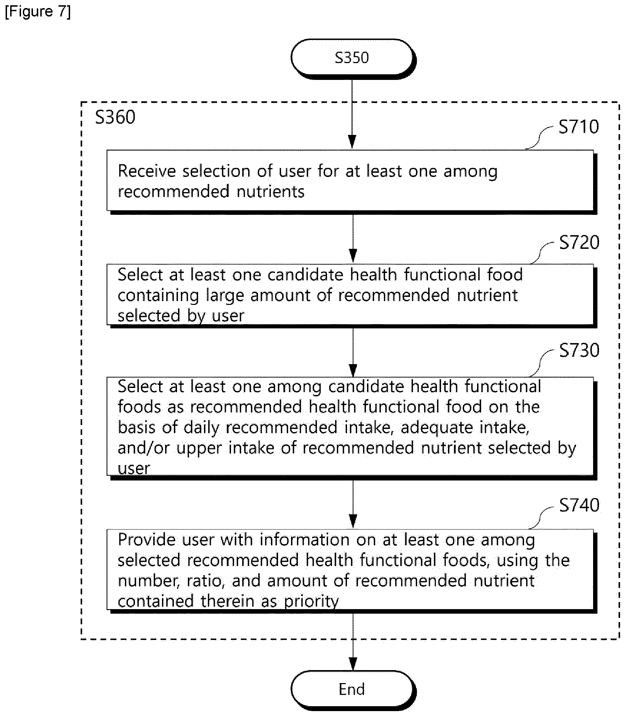

FIG. 7 is a flowchart illustrating an embodiment of step S360 in FIG. 3.

Referring to FIG. 7, the customized healthcare system server 20 may select at least one recommended health functional food on the basis of a daily recommended intake or the like for a recommended nutrient.

First, at step S710, the customized healthcare system server 20 may receive user's selection of at least one among the recommended nutrients selected through step S350 of FIG. 3.

For example, the customized healthcare system server 20 may transmit a list of and/or detailed information on the recommended nutrients to the terminal 10, and receive a user's selection of at least one among the recommended nutrients from the terminal 10 in response thereto.

Subsequently, at step S720, the customized healthcare system server 20 may select at least one candidate health functional food containing a large amount of the recommended nutrient selected by the user.

For example, the customized healthcare system server 20 may acquire information on the health functional food and nutrients contained therein from the database server 30 or a database embedded therein, and select, on the basis of the acquired information, at least one candidate health functional food containing a large amount of the recommended nutrient selected by the user.

Subsequently, at step S730, the customized healthcare system server 20 may select at least one among the candidate health functional foods as a recommended health functional food on the basis of at least one among a daily recommended intake, a daily adequate intake, and a daily upper intake of the recommended nutrient selected by the user.

For example, when the daily recommended intake of the recommended nutrient selected by the user is 100mg, at least one candidate health functional food containing the recommended nutrient as much as the recommended daily intake may be selected as a recommended health functional food.

In addition, for example, when the user selects a first recommended nutrient in advance through step S710, and information on the recommended health functional food containing the first recommended nutrient is provided to the terminal 10 on the basis of the first recommended nutrient, and the user selects the same recommended nutrient again, a candidate health functional food containing an amount corresponding to the remaining recommended intake on the basis of the daily recommended intake of the corresponding recommended nutrient of the user may be selected as a recommended health functional food.

In addition, for example, when the user selects a first recommended nutrient and a second recommended nutrient at the same time, and information on a recommended health functional food has already been provided as described above in relation to the first recommended nutrient, a candidate health functional food that does not contain or contains the first recommended nutrient within the recommended daily intake and contains the second recommended nutrient may be selected as a recommended health functional food.

In an embodiment, at step S740, the customized healthcare system server 20 may provide the user with information on at least one among the selected recommended health functional foods, using at least one among the number of recommended nutrients contained therein, the ratio of each recommended nutrient contained therein, and the amount of each recommended nutrient as a priority.

For example, the customized healthcare system server 20 may be configured to preferentially provide the terminal 10 with information on the health functional food within a predetermined ranking in order of the amount of the recommended nutrients contained therein.

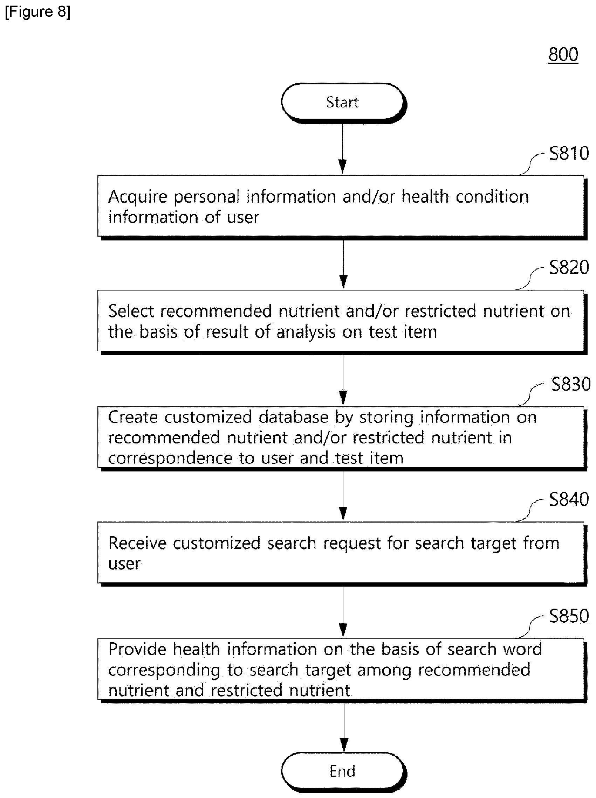

FIG. 8 is a flowchart illustrating an operation method of a customized healthcare system server according to an embodiment of the present invention.

At step S810, the customized healthcare system server 20 may receive personal information and/or health condition information of a user.

In an embodiment, the customized healthcare system server 20 may receive personal information of the user from the terminal 10.

In order to perform step S810, the terminal 10 may be provided with a predetermined user interface for inputting personal information of the user. For example, the user may transfer the input personal information to the customized healthcare system server 20 by activating a corresponding user interface by driving a dedicated application installed in the terminal 10.

In an embodiment, the customized healthcare system server 20 may acquire health condition information of the user from the database server 30 and/or the terminal 10.

Here, the health condition information is at least one piece of information related to an index indicating a degree of health of the user, and the health condition information may include, for example, at least one among medical checkup information, medical treatment information, and lifelog information. The lifelog information may include sleep-related information, eating habit information, life habit information, exercise habit information, living environment information, and the like of the user.

The customized healthcare system server 20 may acquire health condition information of the user from the database server 30 on the basis of the user's identification information.

In an embodiment, the customized healthcare system server 20 may acquire health condition information through a medical questionnaire of a user. That is, the health condition information may be a result determined on the basis of a user's response to the medical questionnaire provided on the terminal 10.

At step S820, the customized healthcare system server 20 may select at least one among recommended nutrients and restricted nutrients on the basis of the health condition information.

In an embodiment, step S820 may be performed through steps S330 to S350 described above with reference to FIG. 3.

In an embodiment, the customized healthcare system server 20 may determine whether or not to select recommended nutrients and/or restricted nutrients on the basis of the analysis result of at least one test item.

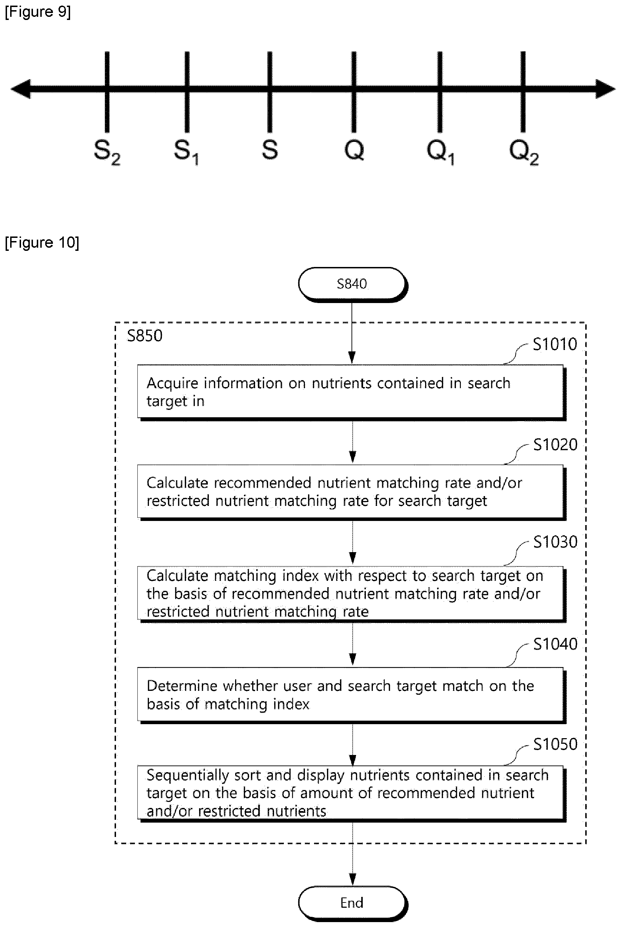

Referring to FIG. 9, the analysis result of the user for each test item may be displayed in a numerical form (i.e., analysis value), and the customized healthcare system server 20 may select recommended nutrients and/or restricted nutrients according to the range to which the analysis value k belongs.

For example, it may be configured not to select recommended nutrients and restricted nutrients when the analysis value k for a certain test item falls within a normal range ($S < k < Q$), and to select recommended nutrients and/or restricted nutrients related to the health item only when the analysis value k is out of the normal range ($k < S, k > Q$).

In an embodiment, the customized healthcare system server 20 may assign a different risk index according to the range to which the analysis value k belongs, and select at least one recommended nutrient and/or restricted nutrient on the basis of the risk index. For example, as shown below in Table 1, the customized healthcare system server 20 may be configured to assign a risk index. Here, the risk index may increase from A to C.

[Table 1]

| Analysis value (k) | Risk index |
| --- | --- |
| $S < k < Q$ (Normal) | None |
| $S1 < k < S, Q < k < Q1$ (Caution) | A |
| $S2 < k < S1, Q1 < k < Q2$ (Dangerous) | B |
| $k < S2, k > Q2$ (Very dangerous) | C |

According to embodiments, the customized healthcare system server 20 may preferentially select recommended nutrients and/or restricted nutrients in relation to a health item of a high-risk index, or select recommended nutrients and/or restricted nutrients only for a health item having a risk index greater than or equal to a reference value. In addition, according to embodiments, the customized healthcare system server 20 may be configured to select a different number of recommended nutrients and/or restricted nutrients according to the risk index.

In addition, according to embodiments, when a specific nutrient corresponds to a recommended nutrient for a first test item and the same nutrient corresponds to a restricted nutrient for a second test item, the customized healthcare system server 20 may compare the risk index of the first test item with that of the second test item, and select a corresponding nutrient as a recommended nutrient or a restricted nutrient on the basis of a test item having a higher risk index. At this point, when the risk indexes are the same, the nutrient may not be selected as a recommended nutrient and a restricted nutrient.

In an embodiment, the customized healthcare system server 20 may calculate a cross risk index between test items, and select recommended nutrients and/or restricted nutrients on the basis of the cross-risk index. For example, the customized healthcare system server 20 may calculate a risk index of "diabetes due to impaired liver function" by cross-analyzing a first blood test value for blood sugar and a second blood test value related to liver function, and select recommended nutrients and/or restricted nutrients on the basis of the risk index.

At step S830, the customized healthcare system server 20 may create a database customized to a user by storing information on the selected recommended nutrients and/or restricted nutrients in correspondence to the user and the test items (or the analysis result of the test items).

Although the customized healthcare system server 20 may create the database by storing the information in an external database server or a cloud server, it is not limited thereto.

At step S840, the customized healthcare system server 20 may receive a user's customized search request for a search target from the terminal 10. Here, the search target includes at least one among a food, a food material, a nutrient, and a health functional food, and the customized search request may include a search word (or keyword) corresponding to such a search target (or referring to the search target).

For example, the user may transmit a customized search request to the customized healthcare system server 20 by inputting a search word corresponding to a search target through the user interface provided on the terminal 10.

At step S850, the customized healthcare system server 20 may provide health information including information on a search target and whether the user and the search target match, on the basis of at least one among the recommended nutrients and the restricted nutrients and the search word.

For example, when the search target is a food, a food material, and/or a health functional food, the customized healthcare system server 20 may determine whether it is appropriate for the user to take the search target by comparing the nutrients contained in the search target with the recommended nutrients and/or the restricted nutrients for the user, and provide information thereon through the terminal 10, together with information on the nutrients contained in the search target.

In addition, for example, when the search target is a nutrient, the customized healthcare system server 20 may detect at least one product (food, food material, and/or health functional food) containing the search target as a nutrient, determine a product appropriate for the user to take among corresponding products by comparing the nutrients contained in the product with recommended nutrients and/or restricted nutrients, and provide information thereon through the terminal 10, together with information on the nutrients contained in the product.

In an embodiment, the customized healthcare system server 20 may further determine whether the search target is appropriate, on the basis of the personal information of the user. For example, the customized healthcare system server 20 may determine that the search target is inappropriate for the user when an ingredient that causes an allergy is contained in the food material, food, or health functional food, which is the search target, according to information on the allergy of the user. In addition, for example, the customized healthcare system server 20 may determine that the search target is inappropriate for the user according to information on the medicine taken by the user when an ingredient that reduces the effect of the medicine is contained in the food material, food, or health functional food, which is a search target, or when the nutrients contained in the medicine taken by the user are overlapped with the nutrients contained in the health functional food, which is a search target, as much as a predetermined proportion or more.

In an embodiment, the operation method 800 may further include the step of providing, by the customized healthcare system server 20, purchase information of a search target or a product containing nutrients of the search target. Accordingly, the user may confirm various brands and/or sellers related to the foods, food materials, and health functional foods corresponding to the search target through the terminal 10, and make a purchase for them. At this point, the purchase information may be provided only when the search target or the product is appropriate for the user more than a predetermined level.

In an embodiment, the operation method 800 may further include the step of collecting information on the consumption pattern of the user, by the customized healthcare system server 20, when a food, a food material, or a health functional food is ordered on the basis of the purchase information provided to the terminal 10.

FIG. 10 is a flowchart illustrating an embodiment of step S850 in FIG. 8.

Referring to FIG. 10, the customized healthcare system server 20 may compare the nutrients contained in the search target with recommended nutrients and/or restricted nutrients, and provide health information on the basis of the comparison. At this point, the search target may be at least one among a food, a food material, and a health functional food.

At step S1010, when a user inputs a search word corresponding to a search target through the terminal 10, the customized healthcare system server 20 may acquire information on the nutrients contained in the search target in correspondence thereto. Information on the contained nutrients may be acquired as the customized healthcare system server 20 searches for information on the ingredient table of the search target from the ingredient DB or the like constructed in the database server 30.

At step S1020, the customized healthcare system server 20 may calculate a recommended nutrient matching rate and/or a restricted nutrient matching rate for the search target.

For example, the customized healthcare system server 20 may calculate the recommended nutrient matching rate and the restricted nutrient matching rate through the following equation, respectively.

$$\text{Recommended nutrient matching rate} = L/N \times 100(\%)$$

$$\text{Restricted nutrient matching rate} = M/N \times 100(\%)$$

Here, L may denote the number of nutrients corresponding to the recommended nutrients among the nutrients contained in the search target, M may denote the number of nutrients corresponding to the restricted nutrients among the nutrients contained in the search target, and N may denote the total number of nutrients contained in the search target.

At step S1030, the customized healthcare system server 20 may calculate a matching index with respect to the search target on the basis of the recommended nutrient matching rate and/or the restricted nutrient matching rate.

For example, the matching index may be calculated by subtracting the restricted nutrient matching rate from the recommended nutrient matching rate. At this point, the higher the matching index, the higher the ratio of the recommended nutrients to the restricted nutrients contained in the search target. However, this method is exemplary and not limited thereto, and when the restricted nutrients matching rate is lower than or equal to a predetermined reference value, various methods such as a method of reflecting only the recommended nutrient matching rate in the matching index may be applied.

At step S1040, the customized healthcare system server 20 may determine whether the user and the search target match on the basis of the matching index. For example, the customized healthcare system server 20 may determine that it is appropriate for the user to take the search target when the matching index is greater than or equal to a predetermined reference value. In addition, for example, the customized healthcare system server 20 may grant a different custom grade according to a range of numerical values to which the matching index belongs, and determine whether the user and the search target match as one of a plurality of custom grades on the basis of this.

At step S1050, the customized healthcare system server 20 may provide the terminal 10 with health information including information on the search target and whether the user and the search target match. For example, information on the search target may include information on the nutrients contained in the search target, and information on whether the search target and the user match may include information on whether or not to recommend the user to take the search target and information on the matching index.

In an embodiment, the customized healthcare system server 20 may sequentially sort the nutrients contained in the search target on the basis of the amounts of the recommended nutrients and/or the restricted nutrients, and display the nutrients on the terminal 10.

In addition, in an embodiment, the customized healthcare system server 20 may compare the amount of the nutrients contained in the search target with the amount of the recommended nutrients and/or the restricted nutrients recommended to take daily, and preferentially sort and display nutrients contained therein more than the recommended amount.

In addition, in an embodiment, the customized healthcare system server 20 may sequentially sort and display the nutrients contained in the search target on the basis of the risk index of test items corresponding to the recommended nutrients and/or the restricted nutrients (i.e., sort in order of nutrients having a high-risk index).

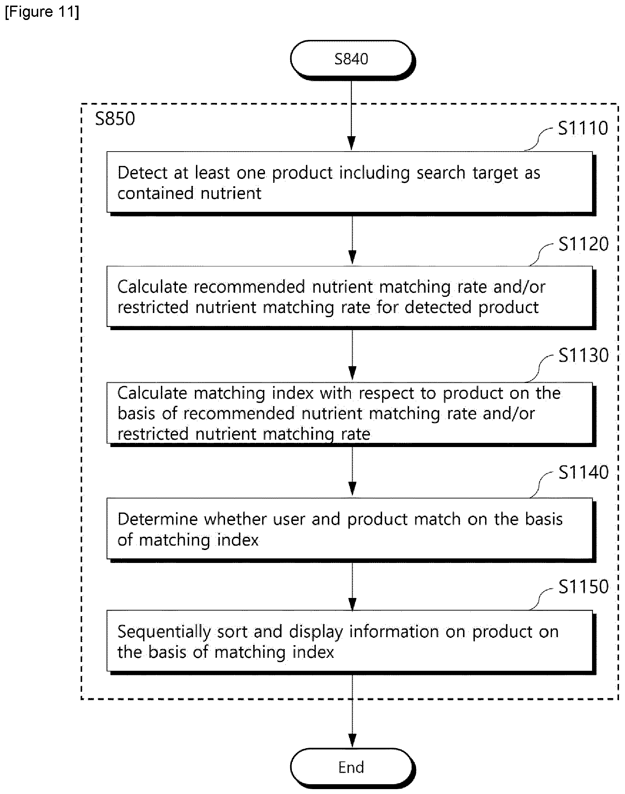

FIG. 11 is a flowchart illustrating an embodiment of step S850 in FIG. 8.

Referring to FIG. 11, the customized healthcare system server 20 may detect at least one product containing a search target as a contained nutrient, compare the nutrients contained in the product with the recommended nutrients and/or the restricted nutrients, and provide health information on the basis of the result of the comparison. At this point, the search target may be a nutrient, and the product may be at least one among a food, a food material, and a health functional food.

At step S1110, when the user inputs a search word corresponding to the search target through the terminal 10, the customized healthcare system server 20 may detect at least one product including the search target as a contained nutrient in response thereto. Such a product may be acquired as the customized healthcare system server 20 searches for a product including a search target as an ingredient from the ingredient DB or the like constructed in the database server 30.

At step S1120, the customized healthcare system server 20 may calculate a recommended nutrient matching rate and a restricted nutrient matching rate for each product on the basis of the nutrients contained in each product and the recommended nutrients and the restricted nutrients of the user.

For example, the customized healthcare system server 20 may calculate a recommended nutrient matching rate and a restricted nutrient matching rate for each product through the following equation in the same manner as described above with reference to FIG. 10.

$$\text{Recommended nutrient matching rate} = L/N \times 100(\%)$$

$$\text{Restricted nutrient matching rate} = M/N \times 100(\%)$$

Here, L may denote the number of nutrients corresponding to the recommended nutrients among the nutrients contained in the product, M may denote the number of nutrients corresponding to the restricted nutrients among the nutrients contained in the product, and N may denote the total number of nutrients contained in the product.

At step S1130, the customized healthcare system server 20 may calculate a matching index between each product and the user on the basis of the recommended nutrient matching rate and/or the restricted nutrient matching rate.

For example, the matching index may be calculated by subtracting the restricted nutrient matching rate from the recommended nutrient matching rate for each product. At this point, the higher the matching index, the higher the ratio of the recommended nutrients to the restricted nutrients contained in the search target. However, this method is exemplary and not limited thereto, and when the restricted nutrients matching rate is lower than or equal to a predetermined reference value, various methods such as reflecting only the recommended nutrient matching rate in the matching index may be applied.

At step S1140, the customized healthcare system server 20 may determine whether the user and the product match on the basis of the matching index. For example, the customized healthcare system server 20 may determine that it is appropriate for the user to take the product when the matching index is greater than or equal to a predetermined reference value. In addition, for example, the customized healthcare system server 20 may grant a different custom grade according to a range of numerical values to which the matching index belongs, and determine whether the user and the product match as one of a plurality of custom grades on the basis of this.

At step S1150, the customized healthcare system server 20 may sequentially sort and display information on the product on the terminal 10 on the basis of the matching index. For example, information on the product may include information on the nutrients contained in the product, the matching index, and information on whether or not to recommend based thereon.

At this point, the customized healthcare system server 20 may sequentially sort and display the nutrients contained in each product on the basis of the amount of recommended nutrients and/or restricted nutrients.

In an embodiment, the customized healthcare system server 20 may sequentially sort the nutrients contained in the product on the basis of the amount of the recommended nutrients and/or the restricted nutrients, and display the nutrients on the terminal 10. In addition, in an embodiment, the customized healthcare system server 20 may sequentially sort and display the nutrients contained in the product on the basis of the risk index of test items corresponding to the recommended nutrients and/or the restricted nutrients (i.e., sort in order of nutrients having a high-risk index).

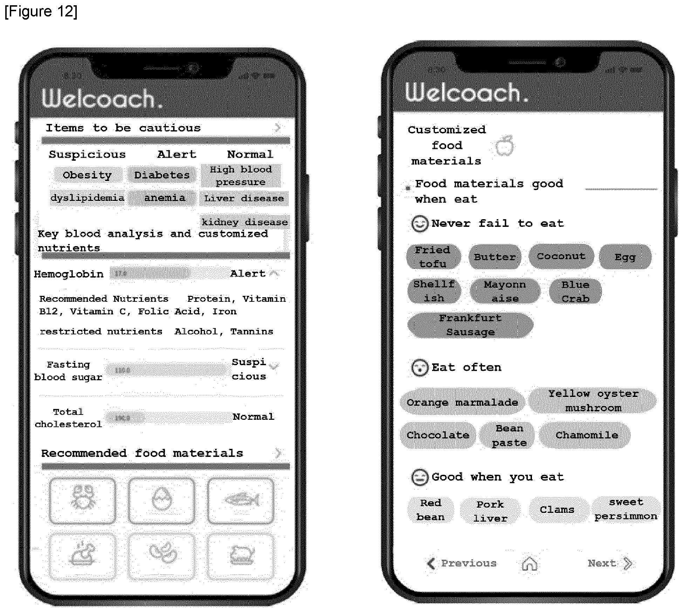

FIGS. 12 to 15 are views for explaining an exemplary operation of a customized healthcare system according to an embodiment of the present invention.

More specifically, FIGS. 12 to 15 exemplarily show a user interface and information provided to the terminal 10 of a user through a dedicated application according to the operation of the customized healthcare system.

First, referring to FIG. 12, the terminal 10 may be provided with information on the risk level (or risk class) related to the test items of the user extracted by the customized healthcare system server 20 on the basis of the medical checkup information and/or medical treatment information of the user, information on the recommended nutrients and restricted nutrients for improving health risk factors related to the test items, and information on the recommended food materials containing a small amount of the restricted nutrients and a large amount of the recommended nutrients.

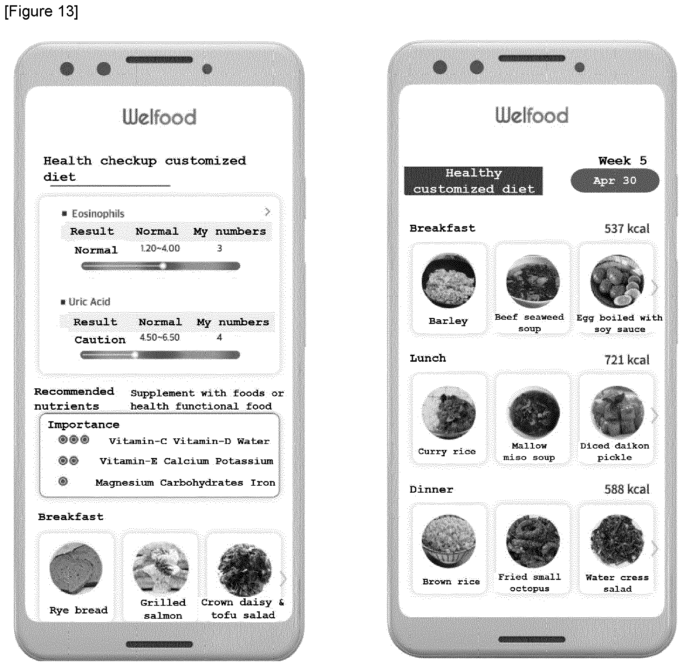

In addition, referring to FIG. 13, the terminal 10 may be further provided with customized diet information of the user selected by the customized healthcare system server 20 on the basis of the recommended nutrients. The corresponding diet may include information on the food or food name, intake amount, calories, and the like.

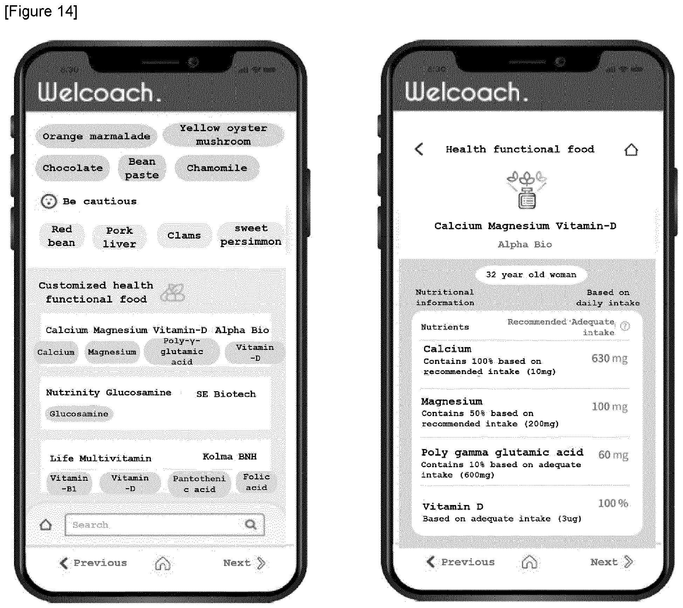

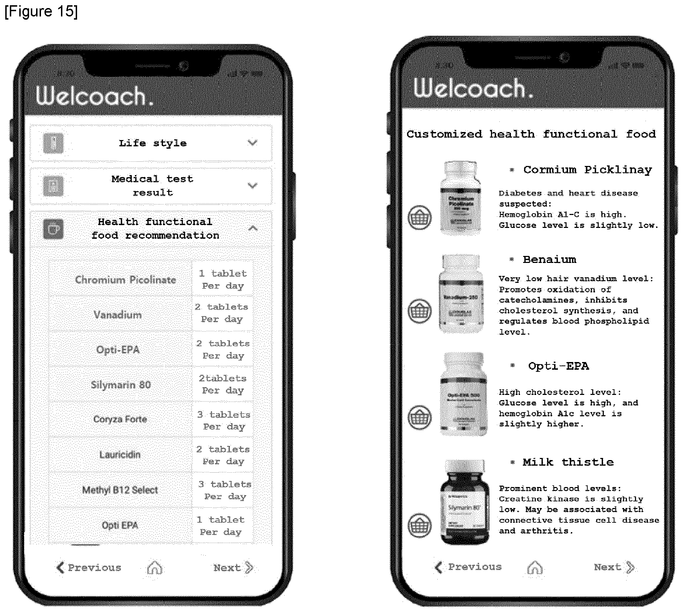

In addition, referring to FIGS. 14 and 15, the terminal 10 may be further provided with information on the recommended health functional food selected by the customized healthcare system server 20 on the basis of the recommended nutrients. Information on the recommended health functional food may include information on the nutrients contained in the food, the amount of the nutrients, the ratio of the nutrients compared to the recommended/sufficient/upper intake, and the number of times, method, and effect of taking the food daily.

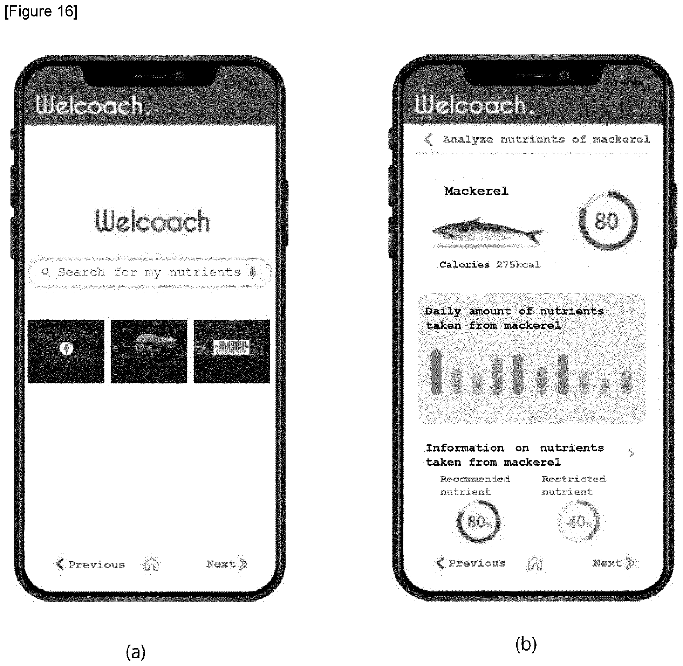

FIG. 16 is a view for explaining an exemplary operation of a customized healthcare system according to an embodiment of the present invention.

More specifically, FIG. 16 exemplarily shows a user interface and information provided to the terminal 10 of a user through a dedicated application according to the operation of the customized healthcare system. Hereinafter, through steps S810 to S830 described above with reference to FIG. 8, it will be described in detail on the assumption that a customized database of the user is created.

First, referring to FIG. 16(a), a user may input a search word corresponding to a search target into the terminal 10 through a user interface for inputting search words. For example, the user may input the name of food material "mackerel" as a search word. When the search word is input, a customized search request may be transmitted from the terminal 10 to the customized healthcare system server 20.

Subsequently, referring to FIG. 16(b), health information generated by the customized healthcare system server 20 in response to the customized search request may be displayed on the terminal 10. As shown in the drawing, basic information of a food material to be searched, contained nutrients, a degree of a search target appropriate to the user (matching score), a recommended nutrient matching rate and a restricted nutrient matching rate of the search target, and the like may be provided to the terminal 10 as health information.

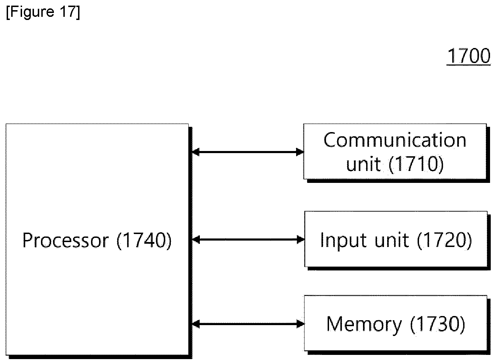

FIG. 17 is a block diagram showing the configuration of a customized healthcare system according to an embodiment of the present invention.

As shown in FIG. 17, the customized healthcare system may include a communication unit 1710, an input unit 1720, a memory 1730, and a processor 1740. However, the components of the customized healthcare system are not limited to the example described above. For example, the customized healthcare system may include more or fewer components than the components described above. In addition, at least two or more among the communication unit 1710, the input unit 1720, the memory 1730, and the processor 1740 may be implemented in the form of one chip.

The communication unit 1710 may transmit and receive signals to and from an external device. The signals transmitted and received to and from the external device may include control information and data. At this point, the external device may include the terminal 10, the database server 30, and the like. The communication unit 1710 may include both wired and wireless communication units. In addition, the communication unit 1710 may receive signals through a wired/wireless channel and output the signals to the processor 1740, and transmit the signals output from the processor 1740 through a wired/wireless channel.

The input unit 1720 may receive various user commands through external handling. To this end, the input unit 1720 may include or connect to one or more input devices. For example, the input unit 1720 may be connected to an interface for various inputs, such as a keypad, a mouse, and the like, to receive user commands. To this end, the input unit 1720 may include an interface such as a Thunderbolt, as well as a USB port. In addition, the input unit 1720 may receive external user commands by including various input devices such as a touch screen, buttons, and the like or in combination with the input devices.

The memory 1730 may store programs and data needed for the operation of the customized healthcare system. In an embodiment, the memory 1730 may store control information or data included in the signals transmitted and received by the customized healthcare system. The memory 1730 may be configured as a storage medium, such as ROM, RAM, hard disk, CD-ROM, or DVD, or a combination of storage media. In addition, the memory 1730 may be provided in plurality. According to an embodiment, the memory 1730 may store programs for performing the operations for the customized healthcare system, which are embodiments of the present invention described above.

The processor 1740 may control a series of processes operated by the customized healthcare system according to an embodiment of the present invention described above. For example, components of the customized healthcare system may be controlled to perform the operation of the customized healthcare system according to an embodiment. The processor 1740 may be provided in plurality, and the processor 1740 may perform the operation of the customized healthcare system by executing a program stored in the memory 1730.

According to an embodiment, the processor 1740 may control to acquire at least one among health checkup information and medical treatment information of the user, extract an analysis result for at least one test item on the basis of at least one among the health checkup information and the medical treatment information, determine a risk level of the user for the test item on the basis of the analysis result, select at least one among the recommended nutrients and/or the restricted nutrients for the user on the basis of the risk level, and provide customized nutritional information to the user on the basis of at least one among the recommended nutrients and the restricted nutrients. Here, the customized nutritional information may include information on at least one among a recommended nutrient, a restricted nutrient, a recommended food material, a recommended menu, a recommended meal pattern, a recommended diet, and a recommended health functional food.

According to an embodiment, the processor 1740 may control to extract an analysis result for at least one blood test item on the basis of at least one among the health checkup information and the medical treatment information.

According to an embodiment, the processor 1740 may control to determine at least one among a risk level for each of test items and a cross-risk level between the test items.

According to an embodiment, the processor 1740 may control to receive personal information of the user. Here, the personal information may include information on at least one among the age, gender, height, weight, allergy, and medication of the user.

According to an embodiment, the processor 1740 may control to derive at least one candidate nutrient for improving health risk factors related to the test item on the basis of the risk level, and select the recommended nutrient by removing at least one among the candidate nutrients on the basis of information on the medication.

According to an embodiment, the processor 1740 may control to derive at least one candidate food material on the basis of at least one among the recommended nutrients and the restricted nutrients, and select the recommended food material by removing at least one among the candidate food materials on the basis of information on the allergy.

According to an embodiment, the processor 1740 may control to provide the user with information on at least one among the recommended food materials, using at least one among the number of recommended nutrients contained therein, the ratio of each recommended nutrient contained therein, and the amount of each recommended nutrient as a priority.

According to an embodiment, the processor 1740 may control to select at least one recommended food material on the basis of at least one among the recommended nutrients and the restricted nutrients, calculate energy consumption of the user on the basis of the information on at least one among the age, sex, height, and weight, and determine at least one among the recommended meal pattern and the recommended diet of the user on the basis of the recommended food material and the energy consumption.

According to an embodiment, the processor 1740 may control to provide the user with information on at least one among the recommended health functional foods selected using at least one among the number of recommended nutrients contained therein, the ratio of each recommended nutrient contained therein, and the amount of each recommended nutrient as a priority.

According to an embodiment, the processor 1740 may control to receive user's selection of at least one among the recommended nutrients, select at least one candidate health functional food containing a large amount of the recommended nutrient selected by the user, and select at least one among the candidate health functional foods as a recommended health functional food on the basis of at least one among a daily recommended intake, a daily adequate intake, and a daily upper intake of the user for the recommended nutrient selected by the user.

According to an embodiment the processor 1740 may receive, from the user, a customized search request including a search word corresponding to at least one search target among a food, a food material, a nutrient, and a health functional food, and provide health information including information on the search target and whether the user and the search target match on the basis of at least one among the recommended nutrients and the restricted nutrients and the search word in response to the customized search request.

According to an embodiment, the processor 1740 may create a customized database by storing information on at least one among the selected recommended nutrients and restricted nutrients in correspondence to the user and the test items.

According to an embodiment, the processor 1740 may control to receive personal information including information on at least one among allergies and medications of the user, and provide health information further based on the personal information.

According to an embodiment, the processor 1740 may acquire information on the nutrients contained in the search target, calculate at least one among a recommended nutrient matching rate and a restricted nutrient matching rate on the basis of the nutrients contained in the search target, the recommended nutrients, and the restricted nutrients, and determine whether the user and the search target match on the basis of at least one among the recommended nutrient matching rate and the restricted nutrient matching rate. Here, the search target may be at least one among a food, a food material, and a health functional food.

According to an embodiment, the processor 1740 may sequentially sort and display the nutrients contained in the search target on the basis of the amount of at least one among the recommended nutrients and the restricted nutrients.

According to an embodiment, the processor 1740 may calculate a matching index between the user and the search target by subtracting the restricted nutrient matching rate from the recommended nutrient matching rate, and determine whether the user and the search target match on the basis of the matching index.

According to an embodiment, the processor 1740 may detect at least one product including the search target as a contained nutrient, calculate a recommended nutrient matching rate and a restricted nutrient matching rate for each product on the basis of the nutrients contained in the product, the recommended nutrients, and the restricted nutrients, calculate a matching index between the user and each of the products by subtracting the restricted nutrient matching rate from the recommended nutrient matching rate, and determine whether the user and the product match on the basis of the matching index. Here, the search target is a nutrient, and the product may include at least one among a food, a food material, and a health functional food.

According to an embodiment, the processor 1740 may sequentially sort and display information on the plurality of products on the basis of the matching index.

Meanwhile, the embodiment described above may be written as a program that can be executed on a computer, and may be implemented in a general-purpose digital computer that executes the program using a computer-readable medium. In addition, the structure of data used in the embodiment described above may be recorded in a computer-readable medium through various means. In addition, the embodiment described above may be implemented in the form of a recording medium including instructions that can be executed by a computer, such as a program module executed by a computer. For example, the methods implemented as a software module or algorithm are codes or program instructions that can be read and executed by a computer, and may be stored in a computer-readable recording medium.

The computer-readable medium may be any recording medium that can be accessed by a computer, and may include volatile and nonvolatile media, removable and nonremovable media. Although the computer-readable medium may include magnetic storage media such as ROM, floppy disk, hard disk, and the like, and optically readable media such as CD-ROM, DVD, and the like, it not limited thereto. In addition, the computer-readable medium may include computer storage media and communication media.

In addition, a plurality of computer-readable recording media may be distributed in computer systems connected through a network, and data stored in the distributed recording media, for example, program instructions and codes, may be executed by at least one computer.

The specific implementations described in the present invention are merely embodiments and do not limit the scope of the present invention in any way. For brevity of the specification, descriptions of conventional electronic components, control systems, software, and other functional aspects of the systems may be omitted.

**Claims**

1. An operation method of a customized healthcare system, the method comprising the steps of:

   acquiring at least one among health checkup information and medical treatment information of a user;
   extracting an analysis result for at least one test item on the basis of at least one among the health checkup information and the medical treatment information;
   determining a risk level of the user for the test item on the basis of the analysis result;
   selecting at least one among the recommended nutrients and the restricted nutrients for the user on the basis of the risk level; and
   providing customized nutritional information to the user on the basis of at least one among the recommended nutrients and the restricted nutrients, wherein
   the customized nutritional information includes information on at least one among the recommended nutrient, the restricted nutrient, a recommended food material, a recommended menu, a recommended meal pattern, a recommended diet, and a recommended health functional food.

2. The method according to claim 1, wherein the step of extracting an analysis result is performed by extracting an analysis result for at least one blood test item on the basis of at least one among the health checkup information and the medical treatment information.

3. The method according to claim 1, wherein the step of determining a risk level for the test item is performed by determining at least one among a risk level for each of test items and a cross-risk level between the test items.

4. The method according to claim 1, further comprising the step of receiving personal information of the user, wherein the personal information includes information on at least one among the age, gender, height, weight, allergy, and medication of the user.

5. The method according to claim 4, wherein the step of selecting at least one among the recommended nutrients and the restricted nutrients includes the steps of:

   deriving at least one candidate nutrient for improving health risk factors related to the test item on the basis of the risk level; and
   selecting the recommended nutrient by removing at least one among the candidate nutrients on the basis of information on the medication.

6. The method according to claim 4, wherein the step of providing customized nutritional information includes the steps of:

   deriving at least one candidate food material on the basis of at least one among the recommended nutrients and the restricted nutrients; and
   selecting the recommended food material by removing at least one among the candidate food materials on the basis of information on the allergy.

**7.** The method according to claim 6, wherein the step of providing customized nutritional information further includes the step of providing the user with information on at least one among the recommended food materials, using at least one among the number of recommended nutrients contained therein, the ratio of each recommended nutrient contained therein, and the amount of each recommended nutrient as a priority.

**8.** The method according to claim 4, wherein the step of providing customized nutritional information includes the steps of:

selecting at least one recommended food material on the basis of at least one among the recommended nutrients and the restricted nutrients;
calculating energy consumption of the user on the basis of the information on at least one among the age, sex, height, and weight; and
determining at least one among the recommended meal pattern and the recommended diet of the user on the basis of the recommended food material and the energy consumption.

**9.** The method according to claim 1, wherein the step of providing customized nutritional information is performed by providing the user with information on at least one among the recommended health functional foods selected using at least one among the number of recommended nutrients contained therein, the ratio of each recommended nutrient contained therein, and the amount of each recommended nutrient as a priority.

**10.** The method according to claim 9, wherein the step of providing customized nutritional information includes the steps of:

receiving user's selection of at least one among the recommended nutrients;
selecting at least one candidate health functional food containing a large amount of the recommended nutrient selected by the user; and
selecting at least one among the candidate health functional foods as a recommended health functional food on the basis of at least one among a daily recommended intake, a daily adequate intake, and a daily upper intake of the user for the recommended nutrient selected by the user.

**11.** The method according to claim 1, further comprising the steps of:

receiving a customized search request from the user, wherein the customized search request includes a search word corresponding to at least one search target among a food, a food material, a nutrient, and a health functional food; and
providing health information including information on the search target and whether the user and the search target match on the basis of at least one among the recommended nutrients and the restricted nutrients and the search word in response to the customized search request.

**12.** The method according to claim 11, further comprising the step of creating a customized database by storing information on at least one among the selected recommended nutrients and restricted nutrients in correspondence to the user and the test items.

**13.** The method according to claim 11, further comprising the step of receiving personal information including information on at least one among allergies and medications of the user, and wherein the step of providing health information is performed further based on the personal information.

**14.** The method according to claim 11, wherein the search target is at least one among a food, a food material, and a health functional food, and the step of providing health information includes the steps of:

acquiring information on the nutrients contained in the search target;
calculating at least one among a recommended nutrient matching rate and a restricted nutrient matching rate on the basis of the nutrients contained in the search target, the recommended nutrients, and the restricted nutrients; and
determining whether the user and the search target match on the basis of at least one among the recommended nutrient matching rate and the restricted nutrient matching rate.

**15.** The method according to claim 14, wherein the step of providing health information further includes the step of sequentially sorting and displaying the nutrients contained in the search target on the basis of the amount of at least one among the recommended nutrients and the restricted nutrients.

16. The method according to claim 14, wherein the step of determining whether the user and the search target match includes the steps of:

calculating a matching index between the user and the search target by subtracting the restricted nutrient matching rate from the recommended nutrient matching rate; and
determining whether the user and the search target match on the basis of the matching index.

17. The method according to claim 11, wherein the search target is a nutrient, and the step of providing health information further includes the steps of:

detecting at least one product including the search target as a contained nutrient, wherein the product includes at least one among a food, a food material, and a health functional food;
calculating a recommended nutrient matching rate and a restricted nutrient matching rate for each product on the basis of the nutrients contained in the product, the recommended nutrients, and the restricted nutrients;
calculating a matching index between the user and each of the products by subtracting the restricted nutrient matching rate from the recommended nutrient matching rate; and
determining whether the user and the product match on the basis of the matching index.

18. The method according to claim 17, wherein the step of providing health information further includes the step of sequentially sorting and displaying information on the plurality of products on the basis of the matching index.

19. A customized healthcare system comprising:

at least one memory for storing a program for performing customized healthcare; and
at least one processor for acquiring at least one among health checkup information and medical treatment information of a user, extracting an analysis result for at least one test item on the basis of at least one among the health checkup information and the medical treatment information, determining a risk level of the user for the test item on the basis of the analysis result, selecting at least one among the recommended nutrients and the restricted nutrients for the user on the basis of the risk level, and providing customized nutritional information to the user on the basis of at least one among the recommended nutrients and the restricted nutrients by executing the program, wherein
the customized nutritional information includes information on at least one among the recommended nutrient, the restricted nutrient, a recommended food material, a recommended menu, a recommended meal pattern, a recommended diet, and a recommended health functional food.

[Figure 1]

[Figure 2]

```
        10                              20                              30
         )                               )                               )

   ┌──────────────┐            ┌──────────────────┐          ┌──────────────────┐
   │   Terminal   │            │    Healthcare    │          │     Database     │
   │              │            │  system server   │          │      server      │
   └──────┬───────┘            └────────┬─────────┘          └────────┬─────────┘
          │                             │                             │
          │                             │  Medical checkup information and
          │  User's personal information(S210)   medical treatment information(S220)
          │─────────────────────────────>│<─────────────────────────────│
          │                             │                             │
```

S230 ─┐ ┌─────────────────────────────────────────┐
      └─┤ Extract analysis result for at least one  │
        │ test item on the basis of medical         │
        │ checkup information and/or medical         │
        │ treatment information                      │
        └─────────────────────────────────────────┘

S240 ─┐
      └─┐ ┌─────────────────────────────────────────┐
        └─┤ Determine risk level of test item on the │
          │ basis of analysis result                  │
          └─────────────────────────────────────────┘

S250 ─┐ ┌─────────────────────────────────────────┐
      └─┤ Select recommended nutrient and/or        │
        │ restricted nutrient on the basis of risk  │
        │ level                                      │
        └─────────────────────────────────────────┘

          │  Customized nutritional information(S260)
          │<─────────────────────────────

[Figure 3]

<u>300</u>

```
                    ┌─────────────┐
                    │    Start    │
                    └──────┬──────┘
                           │
                           ▼                    S310
        ┌──────────────────────────────────────────┐
        │ Receive user's personal information       │
        └──────────────────┬───────────────────────┘
                           │
                           ▼                    S320
        ┌──────────────────────────────────────────┐
        │ Acquire medical checkup information and/or │
        │ medical treatment information of user      │
        └──────────────────┬───────────────────────┘
                           │
                           ▼                    S330
        ┌──────────────────────────────────────────┐
        │ Extract analysis result for at least one  │
        │ test item on the basis of health checkup  │
        │ information and/or medical treatment       │
        │ information                                │
        └──────────────────┬───────────────────────┘
                           │
                           ▼                    S340
        ┌──────────────────────────────────────────┐
        │ Determine risk level of test item on the  │
        │ basis of analysis result                   │
        └──────────────────┬───────────────────────┘
                           │
                           ▼                    S350
        ┌──────────────────────────────────────────┐
        │ Select recommended nutrient and restricted│
        │ nutrient on the basis of risk level        │
        └──────────────────┬───────────────────────┘
                           │
                           ▼                    S360
        ┌──────────────────────────────────────────┐
        │ Provide customized nutritional information │
        │ to user on the basis of recommended       │
        │ nutrient and restricted nutrient           │
        └──────────────────┬───────────────────────┘
                           │
                           ▼
                    ┌─────────────┐
                    │     End     │
                    └─────────────┘
```

[Figure 4]

```
                    ┌─────────────┐
                    │    S340     │
                    └─────────────┘
                           │
┌ ─ ─ ─ ─ ─ ─ ─ ─ ─ ─ ─ ─ ┼ ─ ─ ─ ─ ─ ─ ─ ─ ─ ─ ─┐
│ S350                     │              ⌐ S410   │
│   ┌──────────────────────▼──────────────────┐   │
│   │ Derive candidate nutrient for user on    │   │
│   │ the basis of risk level                   │   │
│   └──────────────────────┬──────────────────┘   │
│                          │              ⌐ S420   │
│   ┌──────────────────────▼──────────────────┐   │
│   │ Select recommended nutrient by removing  │   │
│   │ at least one among candidate nutrients   │   │
│   │ on the basis of information on            │   │
│   │ taken medicine                            │   │
│   └──────────────────────┬──────────────────┘   │
└ ─ ─ ─ ─ ─ ─ ─ ─ ─ ─ ─ ─ ┼ ─ ─ ─ ─ ─ ─ ─ ─ ─ ─ ─┘
                           │
                    ┌──────▼──────┐
                    │    S360     │
                    └─────────────┘
```

[Figure 5]

```
                    ┌─────────────┐
                    │    S350     │
                    └─────────────┘
                           │
┌ ─ ─ ─ ─ ─ ─ ─ ─ ─ ─ ─ ─ ┼ ─ ─ ─ ─ ─ ─ ─ ─ ─ ─ ─┐
│ S360                     │              ⌐ S510   │
│   ┌──────────────────────▼──────────────────┐   │
│   │ Derive at least one candidate food       │   │
│   │ material on the basis of recommended     │   │
│   │ nutrient and/or restricted nutrient      │   │
│   └──────────────────────┬──────────────────┘   │
│                          │              ⌐ S520   │
│   ┌──────────────────────▼──────────────────┐   │
│   │ Select recommended food material by      │   │
│   │ removing at least one among candidate    │   │
│   │ food materials on the basis of            │   │
│   │ information on allergy                     │   │
│   └──────────────────────┬──────────────────┘   │
│                          │              ⌐ S530   │
│   ┌──────────────────────▼──────────────────┐   │
│   │ Provide user with information on at       │   │
│   │ least one among selected recommended     │   │
│   │ food materials, using number, ratio,     │   │
│   │ and amount of recommended nutrient        │   │
│   │ contained therein as priority             │   │
│   └──────────────────────┬──────────────────┘   │
└ ─ ─ ─ ─ ─ ─ ─ ─ ─ ─ ─ ─ ┼ ─ ─ ─ ─ ─ ─ ─ ─ ─ ─ ─┘
                           │
                    ┌──────▼──────┐
                    │     End     │
                    └─────────────┘
```

[Figure 6]

```
        ⟮  S350  ⟯
```

S360                                          S610

Derive at least one recommended food material on the
basis of recommended nutrient and restricted nutrient

                                              S620

Calculate energy consumption of user on the basis of
information on at least one among age, gender, height,
and weight

                                              S630

Determine at least one among recommended meal pattern
and recommended diet on the basis of recommended
food material and energy consumption

```
        ⟮  End  ⟯
```

[Figure 7]

```
                              ( S350 )
```

S360                                                    S710

Receive selection of user for at least one among
recommended nutrients

                                                        S720

Select at least one candidate health functional food
containing large amount of recommended nutrient
selected by user

                                                        S730

Select at least one among candidate health functional
foods as recommended health functional food on the
basis of daily recommended intake, adequate intake,
and/or upper intake of recommended nutrient selected by
user

                                                        S740

Provide user with information on at least one among
selected recommended health functional foods, using the
number, ratio, and amount of recommended nutrient
contained therein as priority

```
                              ( End )
```

[Figure 8]

800

```
      ┌─────────────┐
      │    Start     │
      └─────────────┘
             │
             ▼                                    S810
┌──────────────────────────────────────────────────┐
│ Acquire personal information and/or health condition│
│ information of user                                 │
└──────────────────────────────────────────────────┘
             │
             ▼                                    S820
┌──────────────────────────────────────────────────┐
│ Select recommended nutrient and/or restricted nutrient on│
│ the basis of result of analysis on test item       │
└──────────────────────────────────────────────────┘
             │
             ▼                                    S830
┌──────────────────────────────────────────────────┐
│ Create customized database by storing information on│
│ recommended nutrient and/or restricted nutrient in  │
│ correspondence to user and test item                │
└──────────────────────────────────────────────────┘
             │
             ▼                                    S840
┌──────────────────────────────────────────────────┐
│ Receive customized search request for search target from│
│ user                                                │
└──────────────────────────────────────────────────┘
             │
             ▼                                    S850
┌──────────────────────────────────────────────────┐
│ Provide health information on the basis of search word│
│ corresponding to search target among recommended    │
│ nutrient and restricted nutrient                    │
└──────────────────────────────────────────────────┘
             │
             ▼
      ┌─────────────┐
      │     End      │
      └─────────────┘
```

[Figure 9]

$$S_2 \quad S_1 \quad S \quad Q \quad Q_1 \quad Q_2$$

[Figure 10]

S840

S850

S1010

Acquire information on nutrients contained in search target in

S1020

Calculate recommended nutrient matching rate and/or restricted nutrient matching rate for search target

S1030

Calculate matching index with respect to search target on the basis of recommended nutrient matching rate and/or restricted nutrient matching rate

S1040

Determine whether user and search target match on the basis of matching index

S1050

Sequentially sort and display nutrients contained in search target on the basis of amount of recommended nutrient and/or restricted nutrients

End

[Figure 11]

S840

S850

Detect at least one product including search target as contained nutrient — S1110

Calculate recommended nutrient matching rate and/or restricted nutrient matching rate for detected product — S1120

Calculate matching index with respect to product on the basis of recommended nutrient matching rate and/or restricted nutrient matching rate — S1130

Determine whether user and product match on the basis of matching index — S1140

Sequentially sort and display information on product on the basis of matching index — S1150

End

[Figure 12]

**Welcoach.**

Items to be cautious  >

| Suspicious | Alert | Normal |
|---|---|---|
| Obesity | Diabetes | High blood pressure |
| dyslipidemia | anemia | Liver disease |
| | | kidney disease |

Key blood analysis and customized nutrients

Hemoglobin [___]                    Alert ⌃

Recommended Nutrients   Protein, Vitamin B12, Vitamin C, Folic Acid, Iron

restricted nutrients   Alcohol, Tannins

Fasting blood sugar [___]            Suspi cious ⌄

Total cholesterol [___]              Normal

Recommended food materials  >

---

**Welcoach.**

Customized food materials 🍎

▪ Food materials good when eat

☺ Never fail to eat

( Fried tofu ) ( Butter ) ( Coconut )   ( Egg )

( Shellf ish ) ( Mayonn aise ) ( Blue Crab )

( Frankfurt Sausage )

☺ Eat often

( Orange marmalade ) ( Yellow oyster mushroom )

( Chocolate ) ( Bean paste ) ( Chamomile )

☺ Good when you eat

( Red bean ) ( Pork liver ) ( Clams ) ( sweet persimmon )

< Previous   ⌂   Next >

[Figure 13]

**Welfood**

Health checkup customized diet

- Eosinophils

| Result | Normal | My numbers |
|--------|--------|------------|
| Normal | 1.20~4.00 | 3 |

- Uric Acid

| Result | Normal | My numbers |
|--------|--------|------------|
| Caution | 4.50~6.50 | 4 |

Recommended nutrients — Supplement with foods or health functional food

**Importance**

| | |
|---|---|
| ⊚⊚⊚ | Vitamin-C Vitamin-D Water |
| ⊚⊚ | Vitamin-E Calcium Potassium |
| ⊚ | Magnesium Carbohydrates Iron |

**Breakfast**

Rye bread | Grilled salmon | Crown daisy & tofu salad

**Welfood**

Healthy customized diet — Week 5 — Apr 30

Breakfast — 537 kcal

Barley | Beef seaweed soup | Egg boiled with soy sauce

Lunch — 721 kcal

Curry rice | Mallow miso soup | Diced daikon pickle

Dinner — 588 kcal

Brown rice | Fried small octopus | Water cress salad

[Figure 14]

[Figure 15]

**Welcoach.**

| Life style ∨ |
| Medical test result ∨ |
| Health functional food recommendation ∧ |

| Chromium Picolinate | 1 tablet Per day |
| Vanadium | 2 tablets Per day |
| Opti-EPA | 2 tablets Per day |
| Silymarin 80 | 2tablets Per day |
| Coryza Forte | 3 tablets Per day |
| Lauricidin | 2 tablets Per day |
| Methyl B12 Select | 3 tablets Per day |
| Opti EPA | 1 tablet Per day |

‹ Previous    ⌂    Next ›

**Welcoach.**

**Customized health functional food**

\* **Cormium Picklinay**

Diabetes and heart disease suspected:
Hemoglobin A1-C is high.
Glucose level is slightly low.

\* **Benaium**

Very low hair vanadium level:
Promotes oxidation of catecholamines, inhibits cholesterol synthesis, and regulates blood phospholipid level.

\* **Opti-EPA**

High cholesterol level:
Glucose level is high, and hemoglobin A1c level is slightly higher.

\* **Milk thistle**

Prominent blood levels:
Creatine kinase is slightly low. May be associated with connective tissue cell disease and arthritis.

‹ Previous    ⌂    Next ›

[Figure 16]

(a)

(b)

[Figure 17]

1700

```
                                            ┌─────────────────────┐
                                            │    Communication     │
                                     ◄──────►│    unit (1710)       │
                                            └─────────────────────┘
┌─────────────────────┐                     ┌─────────────────────┐
│                     │                     │                     │
│  Processor (1740)   │◄───────────────────►│  Input unit (1720)  │
│                     │                     │                     │
└─────────────────────┘                     └─────────────────────┘
                                            ┌─────────────────────┐
                                     ◄──────►│   Memory (1730)     │
                                            └─────────────────────┘
```

## INTERNATIONAL SEARCH REPORT

| International application No. |
|---|
| **PCT/KR2021/017264** |

### A. CLASSIFICATION OF SUBJECT MATTER

**G16H 20/60**(2018.01)i; **G16H 50/20**(2018.01)i; **G16H 10/60**(2018.01)i; **G16H 20/10**(2018.01)i; **A61B 5/00**(2006.01)i; **G16H 50/30**(2018.01)i; **A61B 5/107**(2006.01)i

According to International Patent Classification (IPC) or to both national classification and IPC

### B. FIELDS SEARCHED

Minimum documentation searched (classification system followed by classification symbols)

G16H 20/60(2018.01); G06Q 50/22(2012.01); G16H 10/60(2018.01); H04W 4/02(2009.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 헬스케어(healthcare), 건강 검진(medical examination), 진료(diagnosis), 위험도 (risk), 영양 정보(nutrition information)

### C. DOCUMENTS CONSIDERED TO BE RELEVANT

| Category* | Citation of document, with indication, where appropriate, of the relevant passages | Relevant to claim No. |
|---|---|---|
| Y | KR 10-2021-0018619 A (WHATVITA CO., LTD.) 18 February 2021 (2021-02-18) <br> See paragraphs [0009], [0012], [0014], [0040], [0060]-[0062], [0068] and [0072]; claim 1; and figure 6. | 1-19 |
| Y | KR 10-2020-0003590 A (EWHA UNIVERSITY - INDUSTRY COLLABORATION FOUNDATION) 10 January 2020 (2020-01-10) <br> See paragraphs [0033], [0047] and [0052]; and figure 2. | 1-19 |
| Y | KR 10-2014-0070865 A (KYUNGPOOK NATIONAL UNIVERSITY INDUSTRY-ACADEMIC COOPERATION FOUNDATION) 11 June 2014 (2014-06-11) <br> See paragraphs [0032]-[0035]; claims 1-3; and figure 7. | 6-10 |
| Y | JP 2015-194807 A (SEIKO EPSON CORP.) 05 November 2015 (2015-11-05) <br> See paragraphs [0016], [0019]-[0020], [0085]-[0087] and [0110]; claims 1-3; and figure 1. | 11-18 |
| Y | KR 10-2019-0074474 A (GENOPLAN KOREA INC.) 28 June 2019 (2019-06-28) <br> See paragraph [0070]; and figure 1. | 11-18 |

☑ Further documents are listed in the continuation of Box C.   ☑ See patent family annex.

| | |
|---|---|
| \* Special categories of cited documents: <br> "A" document defining the general state of the art which is not considered to be of particular relevance <br> "D" document cited by the applicant in the international application <br> "E" earlier application or patent but published on or after the international filing date <br> "L" document which may throw doubts on priority claim(s) or which is cited to establish the publication date of another citation or other special reason (as specified) <br> "O" document referring to an oral disclosure, use, exhibition or other means <br> "P" document published prior to the international filing date but later than the priority date claimed | "T" later document published after the international filing date or priority date and not in conflict with the application but cited to understand the principle or theory underlying the invention <br> "X" document of particular relevance; the claimed invention cannot be considered novel or cannot be considered to involve an inventive step when the document is taken alone <br> "Y" document of particular relevance; the claimed invention cannot be considered to involve an inventive step when the document is combined with one or more other such documents, such combination being obvious to a person skilled in the art <br> "&" document member of the same patent family |

| Date of the actual completion of the international search | Date of mailing of the international search report |
|---|---|
| **17 March 2022** | **17 March 2022** |

| Name and mailing address of the ISA/KR | Authorized officer |
|---|---|
| **Korean Intellectual Property Office** <br> **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

## INTERNATIONAL SEARCH REPORT

| | International application No. |
|---|---|
| | **PCT/KR2021/017264** |

**C.     DOCUMENTS CONSIDERED TO BE RELEVANT**

| Category* | Citation of document, with indication, where appropriate, of the relevant passages | Relevant to claim No. |
|---|---|---|
| Y | KR 10-2014-0068726 A (WELLTIZEN et al.) 09 June 2014 (2014-06-09)<br>See paragraphs [0046] and [0079]-[0080]; and figure 1. | 13-18 |

**INTERNATIONAL SEARCH REPORT**
Information on patent family members

International application No.

**PCT/KR2021/017264**

| Patent document cited in search report | | | Publication date (day/month/year) | Patent family member(s) | | | Publication date (day/month/year) |
|---|---|---|---|---|---|---|---|
| KR | 10-2021-0018619 | A | 18 February 2021 | KR | 10-2292344 | B1 | 25 August 2021 |
| KR | 10-2020-0003590 | A | 10 January 2020 | KR | 10-2194912 | B1 | 24 December 2020 |
| KR | 10-2014-0070865 | A | 11 June 2014 | None | | | |
| JP | 2015-194807 | A | 05 November 2015 | CN | 104951645 | A | 30 September 2015 |
| | | | | US | 2015-0279235 | A1 | 01 October 2015 |
| KR | 10-2019-0074474 | A | 28 June 2019 | None | | | |
| KR | 10-2014-0068726 | A | 09 June 2014 | US | 2014-0149143 | A1 | 29 May 2014 |

Form PCT/ISA/210 (patent family annex) (July 2019)